# EUROPÄISCHE PATENTSCHRIFT

(11) **EP 0 937 727 B1**
(45) Veröffentlichungstag und Bekanntmachung des Hinweises auf die Patenterteilung: **30.01.2002**
(21) Anmeldenummer: 99103410.9
(22) Anmeldetag: 22.02.1999
(51) Int. Cl.: C07D 501/34, A61K 31/545

(54) **Verfahren zur Herstellung vom löslichen kristallinen Cefuroxim axetil**
Process of preparation of soluble crystalline cefuroxime axetil
Procédé de préparation de céfuroxime axétil cristallin soluble

(30) Priorität: 20.02.1998 DE 19807248
(43) Veröffentlichungstag der Anmeldung: 25.08.1999
(73) Patentinhaber: FAKO ILACLARI A.S., Levend 80650, Istanbul (TR)
(72) Erfinder: Gündüz, Ahmet Halit c/o FAKO ILACLARI A.S., Levend 80650, Istanbul (TR); Bahar, Mehmet c/o FAKO ILACLARI A.S., Levend 80650, Istanbul (TR); Göktepe, Mehmet c/o FAKO ILACLARI A.S., Levend 80650, Istanbul (TR)
(74) Vertreter: Maiwald, Walter, Dr. Dipl.-Chem.

(56) Entgegenhaltungen:
- EP-A- 0 757 991
- DE-A- 2 706 413
- GB-A- 2 145 409
- US-A- 4 562 181
- US-A- 5 063 224

## Beschreibung

Die Erfindung betrifft den Wirkstoff Cefuroxim axetil und insbesondere in Verfahren zur Herstellung einer kristallinen Form des Wirkstoffs, die besser löslich ist als bekannte kristalline Formen von Cefuroxim axetil.

Der Wirkstoff Cefuroxim axetil ist, soweit er für die pharmazeutische Anwendung zugelassen ist, ein Gemisch zweier Diastereomeren. Das pharmazeutisch verwendbare Diastereomerengemisch, um das es bei der vorliegenden Erfindung ausschließlich geht, ist beispielsweise in USP 23, Seite 315/316 ausdrücklich als Produkt definiert, in welchem die beiden Diastereomeren in einem Verhältnis von etwa 1:1 vorliegen.

Gemäß USP 23 wird eine Probe des Diastereomerengemisches chromatographiert. Dem A-Isomer entspricht dann ein Peak r_{A}; dem B-Isomer entspricht ein zweiter Peak r_{B}. Das Diastereomerenverhältnis wird dann gemäß USP 23, Seite 315, rechte Spalte, bestimmt nach der Formel r_{A}/(r_{A} + r_{B}). Dieses Verhältnis muß zwischen 0,48 und 0,55 liegen, damit das Cefuroxim axetil der Monographie entspricht.

Im Anschluß beschreibt USP 23, Seite 316 einen Test für die Bestimmung der Bioverfügbarkeit dieses, aus beiden Diastereomeren etwa im Verhältnis von 1:1 bestehenden amorphen Produktes. Hierzu wird die Löslichkeit des Wirkstoffes aus einer standardisierten Tablettenformulierung in Methanol bestimmt. Zuläßlich sind solche Produkte, bei denen in 15 Minuten mindestens 60% des angegebenen Gehaltes an Wirkstoff und innerhalb von 45 Minuten mindestens 75% des angegebenen Wirkstoffes aufgelöst sind.

(R,S)-Cefuroxim axetil wie in USP 23 definiert (also als Diastereomerengemisch im Verhältnis von etwa 1:1) ist im Stand der Technik seit langem in wenigstens einer kristallinen und in einer amorphen Form bekannt. Die Herstellung dieser kristallinen Form ist beispielsweise in GB-A 15 71 683 und GB-A 21 45 409 beschrieben.

Solches kristalline (R,S)-Cefuroxim axetil zeigt keine ausreichende Bioverfügbarkeit für die Anwendung als Arzneimittel; es erreicht im Test gemäß USP 23 bei weitem nicht die vorgeschriebene Mindestlöslichkeit.

Im Stand der Technik sind auch Verfahren zur Isolation der einzelnen Diastereomeren beschrieben. Beispielsweise gibt DE 27 06 413 nicht nur ein Verfahren an, wie das Isomerengemisch gewonnen werden kann, sondern auch Vorschriften zur Isolation der einzelnen Diastereomeren durch Umkristallisation aus der isomeren Mischung. Beispielsweise läßt sich das Isomere A hiernach aus einer auf 0°C abgekühlten methanolischen Lösung erhalten. Das so erzeugte Produkt ist jedoch nicht rein, sondern enthält noch kleinere Mengen des anderen Diastereomeren. Gemäß Beispiel 1 der DE 27064/3 wird kristallines Cefuroxim a teteil durch Fällung aus Ethylacetat mit Äther, gewonnen.

Gemäß Beispiel 6 von DE 27 06 413 läßt sich das Isomere B anreichern (der Rest ist das A-Isomere) in dem das Ausgangsprodukt, in welchem beide Isomeren etwa im Verhältnis 1:1 vorliegen, mit einer Probe von Isomer A geimpft und bei 0°C einer Kristallisation überlassen wird. Der so erhaltene Feststoff ist hauptsächlich das Isomere A. Aus der Mutterlauge erhält man weiterhin eine zweite Kristallfraktion, die herkömmliches kristallines Diastereomerengemisch im Verhältnis von etwa 1:1 umfaßt.

Die verbliebene Mutterlauge enthält im wesentlich noch das Isomere B. Nach Eindampfen und Auflösung in Acetylacetat lassen sich mehrere Fraktionen gewinnen, die im wesentlichen aus dem Isomeren B bestehen. Eine Behandlung der festen Rückstände mit Ethylacetat-Ether gibt eine Lösung, in der das B- und das A-Isomere etwa im Verhältnis von 70:30 vorliegen.

Aus US 5,063,224 ist eine Gewinnung des R-Isomeren, im Verhältnis von etwa 85:15 im Gemisch mit dem S-Isomeren bekannt. Auch hier macht man sich die bessere Löslichkeit des R-Isomeren zunutze. Nachdem zunächst aus dem Razemat selektiv das S-Isomere abgetrennt wird, kann aus dem so an R-Isomeren aufgereicherten Feststoff das R-Isomere durch Lösung in Ethylacetat und Eindampfen gewonnen werden.

Das Interesse an der Isolierung des R-Isomeren ist bei diesem Stand der Technik durch die Annahme begründet, daß das R-Isomere im Körper langsamer hydrolisiert wird als das S-Isomere und daher eine bessere Bioverfügbarkeit ergeben sollte.

Solche Präparate, bei denen eines der Isomeren in sehr viel größerer Konzentration vorliegt als das andere Isomere, sind jedoch mangels arzneimittelrechtlicher Zulassung nicht von Bedeutung, und auch nicht Gegenstand der vorliegenden Anmeldung, die sich mit einer Verbesserung des bekannten und im Gebrauch befindlichen (R,S)-Wirkstoffes befaßt, wie in USP 23 definiert.

Die weltweit im Gebrauch befindliche Form des Wirkstoffes ist die amorphe Form wie in USP 23 angegeben und wie beispielsweise beschrieben im US-Patent 4 562 181.

Die amorphe, bioverfügbare Form des Wirkstoffs wird herkömmlicherweise aus der kristallinen, im wesentlichen nicht bioverfügbaren Form hergestellt. Dazu wird zunächst die kristalline Form hergestellt, beispielsweise gemäß dem Ausführungsbeispiel 1 der DE-OS 34 27 828, dem Beispiel 2 der europäischen Patentanmeldung EP 0 107 276 oder auch dem Ausführungsbeispiel 2 des schon genannten US-Patents 4 562 181.

Das so erhaltene Produkt wird üblicherweise aus wasserhaltiger Ethylacetatlösung durch Eindampfen bis zur Trockne gewonnen und mit einem Gemisch von Isopropylalkohol und Diisopropylether gewaschen. Das gewaschene Produkt wird in einem geeigneten Lösungsmittel aufgelöst und dann durch Sprühtrocknung in die gewünschte amorphe Form gebracht.

Während diese amorphe Form von (R,S)-Cefuroxim axetil die benötigte Bioverfügbarkeit zeigt, wäre es wünschenswert, stattdessen eine kristalline Form des Wirkstoffes bei vergleichbarer Bioverfügbarkeit einsetzen zu können. Dies nicht nur deshalb, weil grund sätzlich kristalline Formen langfristig stabiler sind als amorphe Produkte, sondern insbesondere auch, weil die Überführung in die amorphe Form durch Sprühtrocknung nicht nur unerwünschten Energieaufwand erfordert, sondern den Wirkstoff auch thermisch nicht unerheblich belastet. Das sprühgetrocknete amorphe Produkt ist häufig gelblich verfärbt, was darauf hindeutet, daß bei der Sprühtrocknung - wenn auch im geringen Umfangchemische Veränderungen auftreten.

Dabei ist allerdings Voraussetzung, daß auch eine solche kristalline Form zulassungsfähig ist, also z.B. den Definitionen gemäß USP 23 entspricht. Das betrifft insbesondere das Verhältnis der Diastereomeren zueinander (das etwa bei 1:1 liegen muß) und die Löslichkeit aus der Tablette gemäß dem Test in USP 23.

Daher ist es also insbesondere nicht möglich, den Anteil an R-Isomeren zu Lasten des Gehaltes an S-Isomeren wesentlich zu erhöhen, wie etwa in US 5,063,224 vorgeschlagen. Das würde zwar im USP-Test die Methanol-Löslichkeit des Isomerengemisches steigern (weil sich das R-Isomere besser in Methanol löst als das S-Isomere), aber das Produkt läge nicht im zulässigen (R,S)-Verhältnis, wäre insgesamt nicht monographiekonform und daher nicht zulasssungsfähig.

In der EP-A1 0 757 991 ist eine kristalline Form von (R,S)-Cefuroxim axetil beschrieben, die ausweislich ihres Röntgenbeugungsdiagramms und ihres IR-Spektrums von den im Stand der Technik bereits früher bekannten, im wesentlichen nicht bioverfügbaren kristallinen Präparaten verschieden ist. Diese, in der EP-A1 0 757 991 als "Beta-Kristalle" bezeichnete Form von (R,S)-Cefuroxim axetil zeigt ein zu höheren Winkeln (°2Θ) verschobenes Röntgenbeugungsspektrum. Während bei der nicht löslichen kristallinen (R,S)-Form, die in der EP-A1 0 757 991 als "Alpha-Kristalle" bezeichnet wird, der Reflex größter Intensität bei einem Winkel von etwa 7,8 liegt, mit dem zweitstärksten Reflex bei einem Winkel von etwa 19,1, wird für die "Beta-Kristalle" der stärkste Reflex bei einem Winkel von 21,3, mit dem zweitstärksten Reflex bei einem Winkel von 16,9 angegeben.

Die "Beta-Kristalle" werden gemäß diesem Stand der Technik aus den "Alpha-Kristallen" dadurch gewonnen, daß diese in Wasser suspendiert und drei Stunden lang bei 40°C gehalten werden.

Da Cefuroxim axetil sich in Wasser praktisch nicht löst, wäre dies wohl nur als eine Rekristallisation in der Feststoffphase zu verstehen.

Nach anderen Beispielen der EP-A1 0757 991 können die "Beta-Kristalle" dadurch gewonnen werden, daß der Wirkstoff in Methanol bei 40°C gelöst und dann durch Zugabe einer großen Menge Wasser ebenfalls bei 40°C und nachfolgendes Abkühlen gefällt wird.

Das Produkt gemäß EP-A1 0 757 991 läßt sich nach der dortigen Vorschrift erzeugen. Die Löslichkeit des Wirkstoffes (aus der tablettierten Form) wie im folgenden beschrieben und bestimmt nach dem Standardverfahren gem. USP 23, S. 316, erreicht jedoch nach 45 Minuten nur etwas mehr als 40 %.

Es wäre demgegenüber sehr wünschenswert, über eine kristalline Form von (R,S)-Cefuroxim axetil zu verfügen, die bis auf den Umstand, daß sie nicht amorph ist, den Anforderungen gemäß USP 23 im wesentlichen entspricht, und also eine sehr viel höhere Löslichkeit als die kristalline Form gemäß EP-A1 0 757 991 zeigt und damit auch eine deutlich größere Bioverfügbarkeit verspricht.

Die Erfindung löst diese Aufgabe, in dem sie ein Verfahren zur Herstellung einer kristallinen Form von (R,S)-Cefuroxim axetil gemäß USP 23 zur Verfügung stellt, die, aus der gleichen tablettierten Form, eine Löslichkeit von mehr als 50% zeigt Die Löslichkeit übertrifft in bevorzugten Ausführungsformen 60% und erreicht in der gegenwärtig am meisten bevorzugten Form praktisch 75% nach 45 Minuten. Damit entspricht die Löslichkeit des erfindungsgemäß herstellbaren kristallinen Cefuroxim axetils im wesentlichen den Anforderungen von z.B. USP 23 an eine pharmazeutisch brauchbare Form des Wirkstoffs.

Die erfindungsgemäß herstellbare kristalline Form des Wirkstoffes enthält die beiden Diastereomeren im gemäß USP 23 vorgeschriebenen Verhältnis, d.i. im Verhältnis zwischen 0,48 und 0,55. Die spezifische optische Drehung (α)_{D} liegt, wiederum in Übereinstimmung mit USP 23, zwischen 37° und 41°.

Die erfindungsgemäß herstellbare kristalline Form des Wirkstoffs ist durch ein Röntgenbeugungs spektrum identifizierbar, das sich sowohl von dem der altbekannten, im wesentlichen unlöslichen kristallinen Form ("Alpha-Kristalle") als auch von dem der "Beta-Kristalle" gemäß EP-A1 0 757 991 unterscheidet.

Die für die Identifikation der erfindungsgemäßen kristallinen Form des Wirkstoffes bedeutsamsten Reflexe liegen (wenn man die Peakhöhe bzw. Intensität betrachtet) bei folgenden Winkeln (°2Θ, wiederum mit einer Genauigkeit der Angabe von ± 0,05):

| **Winkel (°2θ, ± 0,05)** | **Relative Intensität (Peakhöhe) (%)** |
|---|---|
| 7,870 | 100,0 |
| 18,000 | 51,4 |
| 18,069 | 51,6 |
| 19,290 | 93,2 |
| 22,690 | 63,3 |
| 23,771 | 55,1 |
| 23,810 | 54,2 |
| 23,920 | 57,4 |
| 24,130 | 68,8 |
| 24,170 | 67,0 |
| 24,290 | 51,9 |
| 25,090 | 66,1 |
| 26,711 | 71,5 |
| 26,830 | 81,7 |
| 26,860 | 79,8 |

Betrachtet man die Peakfläche, liegen die wichtigsten Intensitäten bei:

| **Winkel (°2θ; ± 0,05)** | **Relative Intensität (Fläche) (%)** |
|---|---|
| 22,69 | 60 |
| 23,81 | 53 |
| 23,92 | 73 |
| 24,15 | 100 |
| 24,30 | 44 |
| 25,10 | 56 |
| 26,8 | 64 |
| 32,27 | 40 |

Die Röntgenbeugungsspektren wurden in üblicher Weise mit Kupfer Kα-Strahlung (Cu 1,54059) gewonnen.

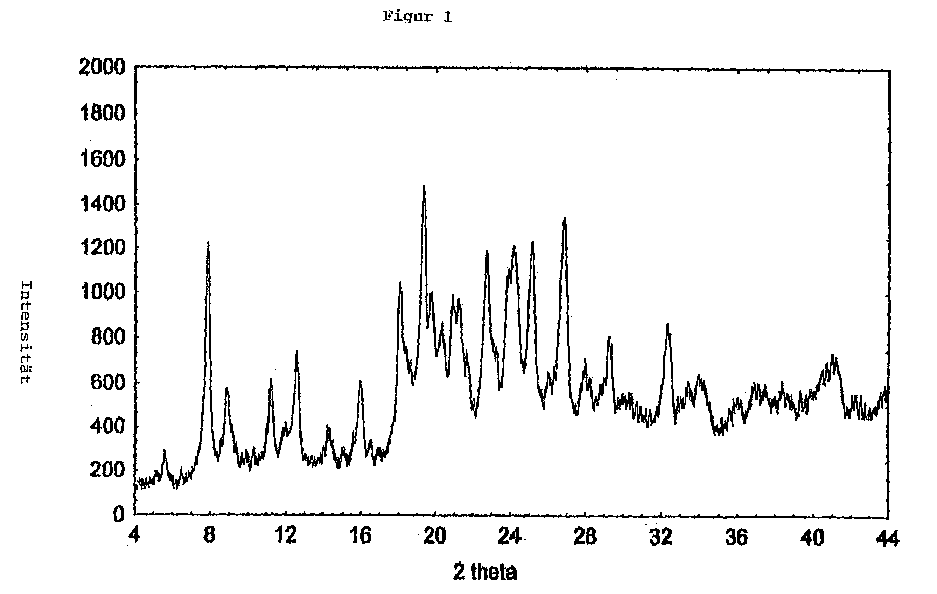

Ein entsprechendes komplettes Beugungsdiagramm ist in Fig. 1 wiedergegeben.

Die erfindungsgemäße hergestellten Präparate sind vollständig kristallin. Die Röntenbeugungsversuche belegen die Abwesenheit von amorphen Anteilen.

Dieses kristalline Cefuroxim axetil läßt sich durch das erfindungsgemäße Verfahren gewinnen, bei dem man kristallines ("Alpha-Kristalle" oder auch "Beta-Kristalle") Das Cefuroxim axetil oder aber amorphes Cefuroxim axetil als Ausgangsprodukt benutzt. Das Ausgangsprodukt ist ein R,S-Gemisch gemäß USP 23, also etwa im R,S-Verhältnis von 1:1. Das Ausgangsprodukt wird, falls nötig unter ausreichendem Erwärmen, in einem Ethylacetät gelöst. Die Lösung wird dann falls nötig eingeengt. Das kristalline Cefuroxim axetil wird aus dieser Lösung durch Zugabe von Diisopropylether bei 30-50°C gefällt.

Wesentlich ist, daß die Verarbeitung, insbesondere im Gegensatz zu der Verfahrensweise gemäß EP-A1 0 757 991, im wesentlichen in Abwesenheit polarer Lösungsmittel erfolgt. Besonders bevorzugt wird in völliger Abwesenheit von Wasser gearbeitet. Stattdessen wird der Wirkstoff aus trockenen Ethylacetat, in dem er in Lösung vorliegt, mit trockenen Diisopropylether gefällt.

Aus dieser Ethylacetatlösung des Ausgangsproduktes wird bei dem gegenwärtig am meisten bevorzugten Herstellungsverfahren das erfindungsgemäße kristalline Produkt dadurch gefällt, daß bei 35°C, langsam der Ether zugesetzt wird. Nach Zugabe des Ethers wird dann zur Vervollständigung der Fällung bei gleicher Temperatur gerührt.

Der erhaltene Feststoff wird abgefiltert und im Vakuum getrocknet; er kann dann verwendet werden.

### Ausführungsbeispiele:

Gemäß dem eingangs genannnten Stand der Technik wurde zunächst kristallines (R,S)-Cefuroxim axetil folgendermaßen hergestellt :

### Beispiel 1

100g Acetylbromid wurden bei Raumtemperatur zu 100ml Methylenchlorid gegeben, welches wasserfreies Kalziumoxid als Trocknungsmittel enthielt. Die Gemisch wurde 45 Minuten lang gerührt. Dann wurden tropfenweise 34,9g Paraldehyd unter Rühren bei Raumtemperatur zugesetzt. Nach weiteren 60 Minuten Rühren wurde das Produkt im Vakuum abdestilliert. Die Umsetzung ergab etwa 110g reines (R,S)-1-Acetoxylethylbromid.

37,5g dieses Produktes wurden bei 0°C zu einer gerührten Suspension von 60g Natrium Cefuroxim in 300ml Dimethylacetamid zugefügt. Die Mischung wurde bei 0°C weitere 90 Minuten gerührt, dann wurde 1,5g Kaliumcarbonat zugefügt. Der Ansatz wurde dann weitere zwei Stunden zwischen +1°C und +3°C gerührt. Dann wurde das Gemisch schnell zu einem Gemisch von 900ml Ethylacetat und 600ml 3%-iger wäßriger Natriumbicarbonatlösung zugefügt. Nach einer Stunde Rühren wurde die organische Schicht abgetrennt, mit 600ml n-HCl-Lösung und dann mit einer Mischung von 50ml gesättigter Kochsalzlösung und 40ml 3%-iger wäßriger Natriumbicarbonatlösung gewaschen. Die organische Phase wurde dann bei Raumtemperatur 30 Minuten lang mit 6g Aktivkohle entfärbt und dann durch ein Kieselguhr-Bett gefiltert. Dann wurde mit 50ml Ethylacetat nachgewaschen.

Die vereinigten Filtrate und Waschflüssigkeiten wurden im Vakuum auf 400ml eingeengt und 30 Minuten lang bei Raumtemperatur gerührt, während die Kristallisation fortschritt. Dann wurde im Vakuum zur Trockene eingedampft. Der feste Rückstand wurde zweimal mit Isopropylalkohol behandelt und im Vakuum getrocknet, dann wurde ein Gemisch von 500ml Isopropylalkohol und Diisopropylether im Volumenverhältnis von 2:3 zugesetzt, um die Kristallisation zu vervollständigen. Der Ansatz wurde dann 30 Minuten lang zwischen 28°C und 32°C gerührt, dann auf unter 5°C abgekühlt und zwischen 0°C und 5°C bei 30 Minuten wiederum gerührt.

Nach Abfilterung des Produktes im Vakuum und Waschen mit 100ml der genannten Isopropylalkohol/Diisopropylether-Mischung wurde das Produkt im Vakuum bei 40°C getrocknet. Man erhielt 56g kristallines Cefuroxim axetil, das hinsichtlich der IR-Daten, dem Röntgenbeugungsdiagramm, der spezifischen optischen Drehung und dem Diastereomerenverhältnis den im Stand der Technik eingegebenen Werten vollkommen entsprach.

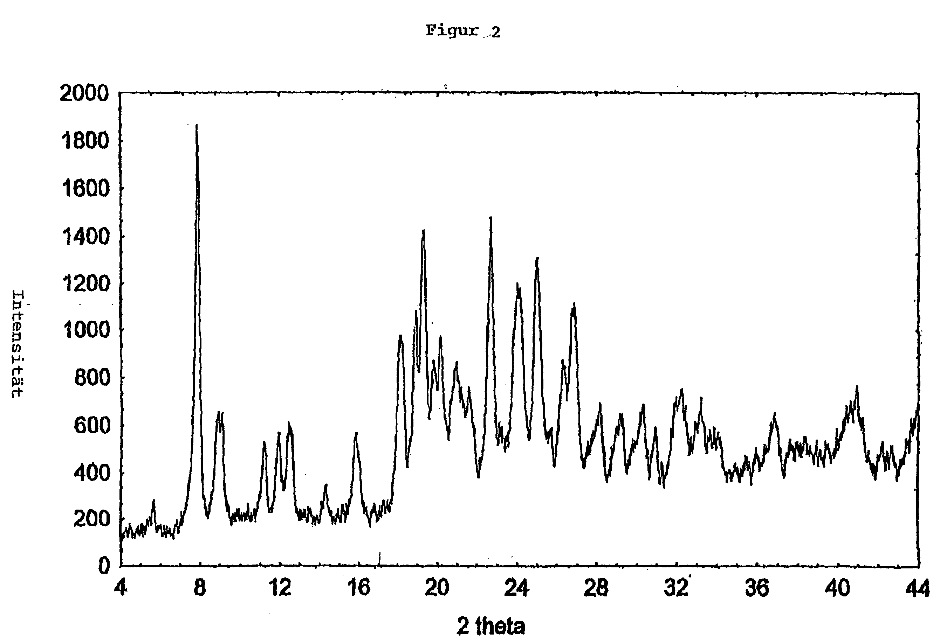

Das Röntgenbeugungsdiagramm dieses Ausgangsmaterials ist in Fig. 2 wiedergegeben.

Aus diesem Ausgangsmaterial wurde das erfindungsgemäße kristalline (R,S)-Cefuroxim axetil folgendermaßen hergestellt:

### Beispiel 2

10g kristallines Cefuroxim axetil gemäß obigem Beispiel 1 wird bei 50°C in 350ml Ethylacetat aufgelöst. Die Lösung wird auf 40°C abgekühlt. Die Mischung wird im Vakuum auf ein Volumen von 175ml eingeengt. Dann werden während 60 Minuten bei 35°C insgesamt 650ml Diisopropylether dazugesetzt. Der Ansatz wird weitere drei Stunden lang bei 35°C gerührt.

Der so erhaltene Feststoffe wird abgefiltert und im Vakuum bei 40°C eingetrocket. Man erhält 7g des erfindungsgemäßen (R,S)-Cefuroxim axetils. Das Produkt entspricht hinsichtlich der Diastereomeren-Zusammensetzung der Vorschrift gemäß USP 23, d.i. es enthält das R- und das S-Diastereomere im Verhältnis von etwa 1:1. Die spezifische optische Drehung des so erhaltenen Produkts liegt zwischen 37° und 41°, wiederum in Übereinstimmung mit USP 23.

### Beispiel 3

Die Herstellung des erfindungsgemäßen Produktes wurde gemäß Beispiel 2 durchgeführt, wobei jedoch als Ausgangsmaterial amorphes (R,S)-Cefuroxim axetil gemäß USP 23 verwendet wurde. Das Ausgangsprodukt stammte aus der Produktion der Anmelderin.

In völlig gleicher Weise wurde erfindungsgemäßes kristallines (R,S)-Cefuroxim axetil erhalten; die spezifische optische Drehung, das Diastereomerenverhältnis und das Röntgenbeugungsspektrum entsprachen denen des Produktes von Beispiel 2.

### Vergleichsbeispiel

Zum Vergleich wurde gemäß der Vorschrift in US 5,063,224 im wesentlichen reines R-Cefuroxim axetil und im wesentlich reines S-Cefuroxim axetil hergestellt. Hierzu wurde amorphes (R,S)-Cefuroxim axetil gemäß USP 23 aus der Produktion der Anmelderin, mit einem Diastereomerenverhältnis von etwa 1:1, in Methanol bei 50° aufgelöst und für eine Stunde gerührt, um das stärker methanollösliche R-Isomer herauszulösen. Das Rühren wurde bei 25°C mehrere Stunden fortgesetzt, dann wurden flüssige und feste Phasen durch Filtration getrennt. Nach Abdampfen im Vakuum wurde aus dem Filtrat hoch angereichertes R-Cefuroxim axetil erhalten, während der Filterkuchen im wesentlich aus dem S-Isomer bestand. Das R-Isomere wurde dann gemäß Beispiel 4 von US 5,063,224 weiter aufgearbeitet. Dementsprechend wurde die Acetonlösung mit Wasser verdünnt und bei 5°C mehrere Tage der Kristallisation überlassen, um das Anhydrat des R-Isomers zu gewinnen. Entsprechend wurde durch Zufügen einer Acetonlösung des R-Isomeren zu Wasser das R-Cefuroxim axetil Hemihydrat erhalten.

Das so erhaltene R-Cefuroxim axetil Anhydrat zeigte eine spezifische optische Drehung (α)_{D} von +18,3°. Die Bestimmung des Diastereomerenverhältnisses (gemäß HPLC, wie oben angegeben) ergab einen Wert von 0,11. Das Produkt bestand also zu etwa 90% aus dem R-Isomer.

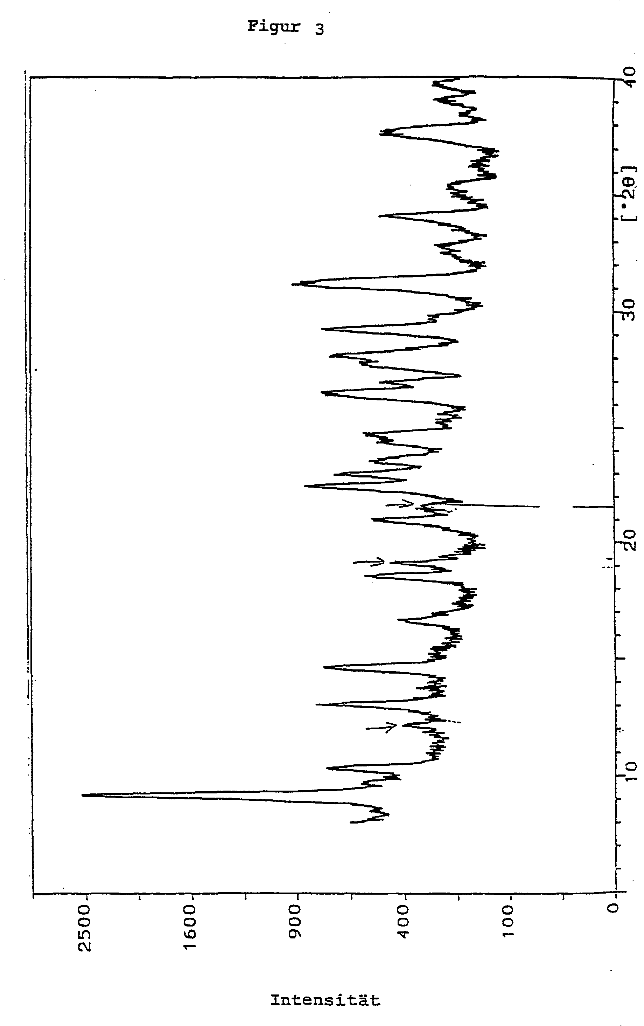

Das entsprechende Röntgenbeugungsdiagramm entspricht den Werten, die in US 5,063,224 angegeben sind, und unterscheidet sich von dem Röntgenbeugungsdiagramm des erfindungsgemäßen Produktes insbesondere durch zusätzliche Peaks bei 2θ = 12,150; 19,135 und 21,650. Die anderen Peaks sind verschoben, bzw. zeigen deutlich unterschiedliche Intensitäten. Das Röntgenbeugungsdiagramm ist als Fig. 3 beigefügt.

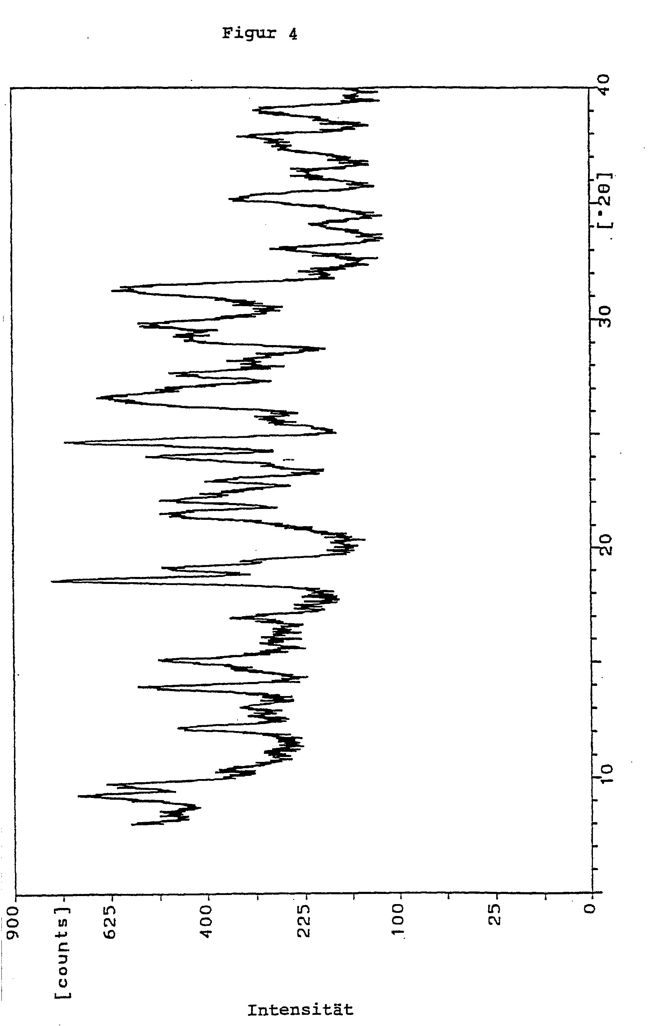

Das R-Cefuroxim axetil Hemihydrat zeigt eine spezifische optische Drehung (α)_{D} von 11,8° und ergab experimentell ein Diastereomerenverhältnis gemäß oben genannter Formel (auf der Grundlage der genannten HPCL-Bestimmung) von 0,05. Das Produkt bestand also zu etwa 95% aus dem Hemihydrat des R-Isomer. Das Röntgenbeugungsdiagramm des Hemihydrats ist als Fig. 4 beigefügt.

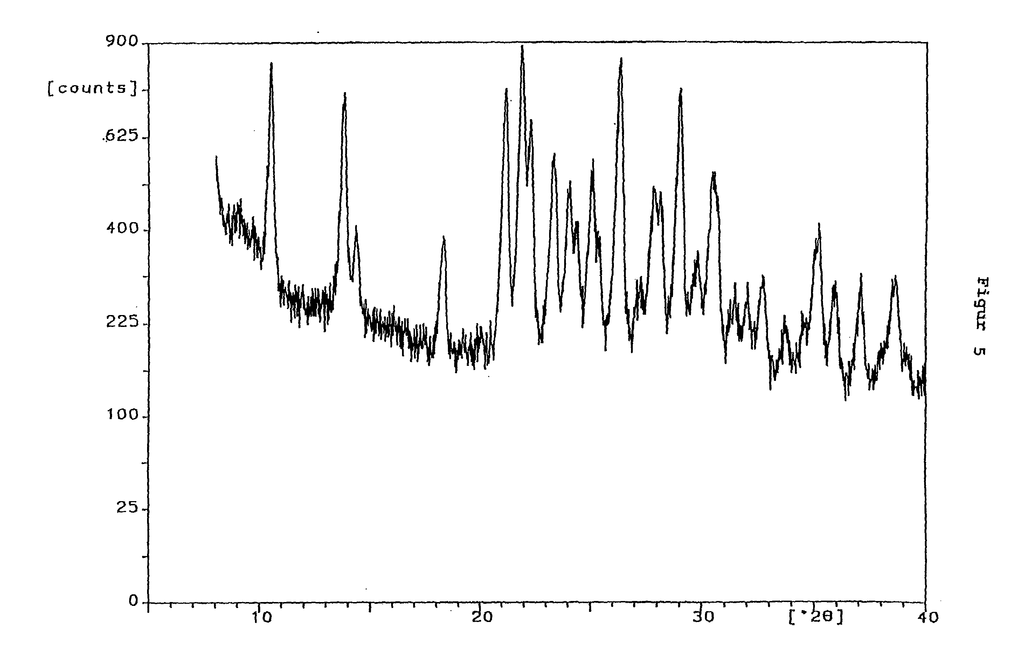

Das so erzeugte S-Isomer zeigte eine spezifische optische Drehung (α)_{D} von +58° und ergab experimentell wie beschrieben ein Diastereomerenverhältnis bei 0,87. Dieses Produkt bestand also zu etwa 87% aus dem S-Isomeren. Das entsprechende Röntgenbeugungsdiagramm ist als Fig. 5 beigefügt.

Wie diese Experimente zeigen, entspricht das Röntgenbeugungsdiagramm des erfindungsgemäßen Produktes weder den einzelnen Isomeren, noch einem Gemisch der R-. und S-Isomeren in den jeweiligen kristallinen Formen. Aus dem Vergleich der spezifischen optischen Drehungen, der Bestimmung der Diasteomerenverhältnisses und dem Vergleich der Röntgenbeugungsdiagramme ergibt sich vielmehr, daß es sich bei dem erfindungsgemäßen Produkt um eine eigene, von R- und S-Form unterschiedene Erscheinungsformen handelt.

Aus dem Vergleich der Röntgenbeugungsdiagramme ergibt sich weiterhin, daß es sich auch im Vergleich mit den bekannten "α-Kristallen" und "β-Kristallen" beider Erfindungen um eine weitere, andere Erscheinungsform des (R,S)-Cefuroxim axetils handelt.

Das wird bestätigt durch die Löslichkeitsverhältnisse, beim Standardversuch gemäß USP 23, die die Erfindung deutlich sowohl von den bekannten α- bzw. β-Kristallen der (R,S)-Form, als auch von den mehr oder weniger reinen Diastereomeren unterscheiden. Ein Gemisch von R- und S-Form etwa im Verhältnis 1:1 wäre, sofern es kristallin vorliegt, natürlich nicht in dem Maße löslich wie dies experimentell für die Erfindung gefunden wird.

### Beispiel 4

Zum Vergleich der Löslichkeiten wurde nach der Herstellungsvorschrift in EP-A1 0 757 991 eine ausreichend große Probe der "Beta-Kristalle" erzeugt und anhand der in dieser Druckschrift angegebenen physikochemischen Parameter identifiziert.

Dann wurden mit dem so gewonnenen Wirkstoff einerseits, dem erfindungsgemäßen Wirkstoff andererseits Tabletten hergestellt, und zwar nach folgendem Verfahren: 305 mg kristallines Cefuroxim axetil wurden mit 50 mg Ac disol, 50 mg Avicel pH 105, 7,5 mg Aerosil 200, 2,5 mg Magnesiumstearat und 7,5 Explotab trocken vermischt. Das Gemisch wurde trocken verpreßt und granuliert. Das so erhaltene Granulat wurde mit 7,5 mg Explotab und 7,5 mg Natriumlaurylsulfat vermischt und das erhaltene Gemisch wurde dann zu Tabletten gepreßt.

Mit den so erhaltenen Tabletten wurde jeweils der Löslichkeitstest gemäß USP 23 durchgeführt. Nach 45 Minuten wurde jeweils der Grad der Auflösung des Wirkstoffes bestimmt. Bei den Tabletten mit dem Wirkstoff gemäß EP-A1 0 757 991 ergab sich eine Löslichkeit von 41,3 %. Bei den erfindungsgemäßen Tabletten lag die Löslichkeit bei 73,0 %.

## Patentansprüche

1. Verfahren zur Herstellung von bioverfügbarem (R,S)-Cefuroxim axetil in kristalliner Form, bei dem man kristallines oder amorphes (R,S)-Cefuroxim axetil als Ausgangsprodukt, gegebenenfalls unter ausreichendem Erwärmen, in Ethylacetat löst, dann gegebenenfalls einengt, und das kristalline bioverfügbare Cefuroxim axetil durch Zugabe von Düsopropylether bei 30-50°C ausfällt.

2. Verfahren nach Anspruch 1, bei dem die Fällung bei 35°C erfolgt.

## Claims

1. A process for the production of bioavailable (R,S)-cefuroxime axetil in crystalline form, wherein crystalline or amorphous (R,S)-cefuroxime axetil as a starting material, is dissolved in ethyl acetate, optionally with sufficient heating, optionally followed by concentrating to a smaller volume, and the crystalline bioavailable cefuroxime axetil is precipitated by adding diisopropylether at 30-50° C.

2. Process according to claim 1, wherein the precipitation is carried out at 35°C.

## Revendications

1. Procédé de fabrication de (R,S)-céfuroxime axetil biologiquement disponible sous forme cristalline dans lequel le produit de base, de la (R,S)-céfuroxime axetil cristalline ou amorphe, est dissous dans de l'ester acétique, le cas échéant en le chauffant, et qui est concentré par la suite et dans lequel la céfuroxime axetil cristalline biologiquement disponible précipite sous addition de diisopropyléther à une température de 30-50°C.

2. Procédé selon la revendication 1 **caractérisé en ce que** la précipitation se fait à une température d'environ 35°C.
